# EUROPEAN PATENT SPECIFICATION

(11) **EP 2 773 630 B1**
(45) Date of publication and mention of the grant of the patent: **14.09.2016**
(21) Application number: 12779083.0
(22) Date of filing: 02.11.2012
(51) Int. Cl.: C07D 307/33

(54) **PROCESS FOR THE PRODUCTION OF HYDROCARBONS**
VERFAHREN ZUR HERSTELLUNG VON KOHLENWASSERSTOFFEN
PROCÉDÉ DE PRODUCTION D'HYDROCARBURES

(30) Priority: 03.11.2011 WO PCT/EP2011/005547
(43) Date of publication of application: 10.09.2014
(73) Proprietor: Haldor Topsøe A/S, 2800 Kgs. Lyngby (DK)
(72) Inventor: HOLM, Martin Spangsberg, GB-OX4 1BE, Oxford (GB); STUMMANN, Hanne Zingler, DK-2300 Copenhagen S (DK); TAARNING, Esben, DK-2000 Frederiksberg (DK)
(74) Representative: Haldor Topsøe A/S
(86) International application number: PCT/EP2012/071679
(87) International publication number: WO 2013/064610

(56) References cited:
- EP-A2- 1 240 941
- WO-A2-2008/151269
- ZHENG H Y ET AL: "Towards understanding the reaction pathway in vapour phase hydrogenation of furfural to 2-methylfuran", JOURNAL OF MOLECULAR CATALYSIS A: CHEMICAL, ELSEVIER, AMSTERDAM, NL, vol. 246, no. 1-2, 1 March 2006 (2006-03-01), pages 18-23, XP025156095, ISSN: 1381-1169, DOI: 10.1016/J.MOLCATA.2005.10.003 [retrieved on 2006-03-01]
- HAYES DANIEL J ET AL: "The Biofine process - production of levulinic acid, furfural, and formic acid from lignocellulosic feedstocks", BIOREFINERIES--INDUSTRIAL PROCESSES AND PRODUCTS,, vol. 1, 1 January 2006 (2006-01-01), pages 139-164, XP009121196,
- RAO R S ET AL: "FURFURAL HYDROGENATION OVER CARBON-SUPPORTED COPPER", CATALYSIS LETTERS, SPRINGER NEW YORK LLC, UNITED STATES, vol. 60, 1 January 1999 (1999-01-01), pages 51-57, XP002469199, ISSN: 1011-372X, DOI: 10.1023/A:1019090520407

## Description

The present invention relates to a process for the production of hydrocarbons, particularly liquid hydrocarbons from biomass derived sugars. More particularly, the invention relates to the production of gamma-valerolactone (GVL) by heterogeneously catalyzed hydrogenation of furfural or furfuryl alcohol, which can be derived from five-carbon sugars. The conversion of furfural or furfuryl alcohol to GVL is conducted in the gas phase and in one single step.

Gamma-valerolactone (GVL) is one of the most interesting platform chemicals within the biomass conversion community since it has been demonstrated that it for example can be converted to hydrocarbons in good yields and of the same quality of hydrocarbons produced according to established petroleum based processes. This brings closer the goal of efficiently and economically producing fuels, particularly liquid fuels such as gasoline and diesel, from biomass.

Furfural is the direct dehydration product of C₅-sugars and has been produced since the 1920'ies on an industrial scale (recently ∼280.000 t/year) from pentose rich substrates such as corncobs and sunflower.

Currently there are commercially viable biomass conversion plants from which furfural is produced. One such process is the so-called Biofine Process according to which bulk lignocellulose is converted into chemical products such as levulinic acid, formic acid (product stream from C6-sugars), furfural (product stream from C5-sugars), and ligneous char.

Prior art dedicated to the use of furfural as substrate deal predominantly with selective hydrogenation of furfural to furfuryl alcohol or 2-methyl furan. Most (∼60%) of furfural produced is converted into furfuryl alcohol which has numerous uses, including conversion into levulinic acid.

It is also known to produce GVL from C6-sugars. According to this route GVL is derived from 5-(hydroxymethyl)furfural (HMF) instead of furfural (a C₅-sugar derivative). HMF is first converted to levulinic acid and formic acid. Levulinic acid can then be converted via catalytic hydrogenation to GVL.

Patent application WO-A-2010151343 discloses a process where cellulose is decomposed to yield a product mixture comprising levulinic acid and formic acid and then the levulinic acid/formic acid solution is converted to GVL over a Ru/C-catalyst. GVL can be further converted to a mixture of n-butenes which can be subjected to isomerisation and oligomerization to yield olefins in the gasoline, jet and diesel fuel ranges.

Patent application WO-A-08151269 discloses a method for converting bio-oil (which is defined as the product liquid from fast pyrolysis of biomass and which can be a single phase or multiphase liquid) in the presence of hydrogen over a catalyst comprising Ru or Pd. Here a liquid phase method for furfural hydrogenation is disclosed by providing a liquid comprising furfural, guaiacol or substituted guaiacol, providing hydrogen and reacting them over a catalyst comprising Ru or Pd. Furfural is converted to i.a. tetrahydrofuran-methanol (THF-MeOH) and 2-methyl- tetrahydrofuran (MTHF). Furfural is to a lesser extent converted to GVL. Hence, this citation discloses the conversion of furfural to GVL in the liquid phase by the use of noble catalysts.

A known multi-step route of making GVL from furfural, and thereby from C5-sugars, is by converting furfural to furfuryl alcohol, then converting it in an alcohol solvent such as butanol to butyl levulinate using an acidic catalyst, and finally reducing the butyl levulinate to GVL in a hydrogenation step.

The abovementioned conversion of furfuryl alcohol to levulinate intermediates is necessarily conducted in the liquid phase with the aid of a suitable homogeneous or solid acidic catalyst. The reduction of levulinic acid forming GVL can subsequently be done either in the liquid or gas phase.

We have now found a selective and direct route for obtaining GVL from furfural or furfuryl alcohol by conducting the conversion of furfural and/or furfuryl alcohol directly to GVL in the gas phase and using a non-noble metal hydrogenation catalyst. The invention is as recited by the following features which correspond to claims 1-12 of this disclosure.

### Features of the invention:

1. Process for the production of gamma-valerolactone (GVL) by conversion in the gas phase of a stream comprising furfural or a stream comprising furfuryl alcohol in the presence of a heterogeneous catalyst comprising copper and alumina in the form of a copper-alumina (Cu-Al) spinel.
2. Process according to feature 1 wherein the stream comprising furfural or stream comprising furfuryl alcohol is co-fed with water.
3. Process according to features 1 or 2, wherein the copper-alumina spinel is a precipitated stoichiometric Cu-Al spinel (CuAl₂O₄).
4. Process according to any of features 1-3, wherein the catalyst is prepared by a co-precipitation method comprising the use of a Cu and Al solution.
5. Process according to feature 4, wherein the atomic ratio Cu²⁺/Al³⁺ in the Cu and Al solution is in the range 1/9 to 7/3 and the co-precipitation is conducted together with a basic solution.
6. Process according to any of features 1 to 5, wherein the process is conducted at a reaction temperature in the range 100 to 300°C.
7. Process according to any of features 1 to 5, wherein the process is conducted at a reaction temperature in the range 120-190°C.
8. Process according to any of features 1 to 5, wherein the process is conducted at a reaction temperature in the range 160-180°C.
9. Process according to any of features 1 to 8, wherein the reaction pressure is in the range 1-30 atm.
10. Process according to any of features 1 to 8, wherein the reaction pressure is in the range 10-25 atm.
11. Process according to any of features 1 to 10, wherein furfural is derived from C5-sugars.
12. Process according to any of features 1 to 11 further comprising converting GVL to liquid hydrocarbons.

In a first aspect of the invention there is provided a process for the production of GVL and use of GVL for the production of liquid hydrocarbons as recited by features 1-12, corresponding to claims 1-12.

It would be understood that the conversion in the gas phase is conducted in the presence of hydrogen.

Preferably, the process is conducted in a fixed bed reactor.

Either stream in the general embodiment of feature 1 may comprise furfural and furfuryl alcohol.

Preferably the stream comprising furfural contains at least 5 wt% furfural, more preferably 5-10 wt% furfural, even more preferably 20-90 wt% furfural. Other constituents of the stream may include furfuryl alcohol or water. A suitable composition of the furfural stream is for instance 100 wt% furfural which then is used in vapour (gas) form when passed over the heterogeneous catalyst. Another suitable composition is a stream containing 5-10 wt% furfural in water, such as 7 wt% furfural in water, which is then used in vapour (gas) form when passed over the heterogeneous catalyst.

Preferably the stream comprising furfuryl alcohol contains at least 10 wt% furfuryl alcohol, more preferably 20-90 wt% furfuryl alcohol. Other constituents of the stream may include furfural or water.

The amount of water in the stream comprising furfural or furfuryl alcohol is preferably up to 95 wt% water, more preferably 50-70 wt% water. In particular, we have also found that it is desirable to conduct the process with an azeotropic mixture of furfural and water. Hence, in a particular embodiment of the invention in connection with one of the above or below embodiments, the amount of water in the stream comprising furfural contains 65 wt% water and 35 wt% furfural. This is the azeotropic composition of furfural and water.

According to the invention the hydrogenation catalyst is a copper alumina spinel, preferably a stoichiometric Cu-Al spinel. By stoichiometric Cu-Al spinel is meant CuAl₂O₄ having a composition 35.0 wt% Cu and 29.7 wt% of Al with the balance being oxygen.

The copper alumina (Cu-Al) catalyst is preferably prepared by a co-precipitation method. More preferably the atomic ratio Cu²⁺/Al³⁺ in the Cu-Al solution is in the range 1/9 to 7/3 and the co-precipitation is conducted together with a basic solution, such as a solution containing NaOH and Na₂CO₃. Particularly, where the catalyst is a Cu-Al spinel the atomic ratio Cu²⁺/Al³⁺ in the Cu-Al solution is 1/2.

In a preferred embodiment of the invention the process is conducted with a feed stream of furfural at a reaction temperature of 200°C, reaction pressure of 20 atm and WHSV (weight hour space velocity) of 1 g furfural/g catalyst/h. This enables the direct conversion of furfural into a product containing 10 wt% GVL.

In another preferred embodiment of the invention the process is conducted with a feed stream of furfural at a reaction temperature of 180°C, reaction pressure of 1 atm and WHSV (weight hour space velocity) of 0.08-0.09 g furfural/g catalyst/h. This enables the direct conversion of furfural into a product containing 21 wt% GVL.

According to the invention, the hydrogenation of furfural or furfuryl alcohol to GVL is conducted in one single step, without requiring first to isolate the alkyl levulinate/levulinic acid.

Without being bound by any theory, it is believed that GVL according to the present invention is obtained by isomerisation of furfuryl alcohol followed by hydrogenation/dehydrogenation.

The invention enables also a cheaper production of GVL and thereby downstream products such as liquid hydrocarbons because the use of noble catalysts such as palladium in the GVL production step is avoided. Further, rather than resorting to homogeneous catalysis or liquid phase reactions the conversion is performed by heterogeneous catalysis conducted in the gas phase.

Currently GVL can be produced from levulinic acid which is a product of C6-sugars. Furfural, as a product of C5-sugars, enables therefore to greatly simplify downstream upgrade of the products, since the GVL product of both C5 and C6-sugars can be further processed as one single combined stream or sold as one. The economics of the biomass conversion process, for instance the Biofine Process is thus significantly enhanced.

In a second aspect the invention encompasses also the use of the copper alumina catalyst according to the invention for the production of gamma-valerolactone (GVL) by gas phase hydrogenation of furfural or furfuryl alcohol. Preferably, the furfural or furfuryl alcohol is co-fed with water. The mixture is then passed in its vapour (gas) form through the heterogeneous catalyst.

As described above, the catalyst is a Cu-Al spinel, preferably a stoichiometric Cu-Al spinel. By stoichiometric Cu-Al spinel is meant CuAl₂O₄ having a composition 35,0 wt.% Cu and 29,7 wt.% of Al with the balance being oxygen.

The copper alumina catalyst is preferably prepared by a co-precipitation method. More preferably the atomic ratio Cu²⁺/Al³⁺ in the Cu-Al solution is in the range 1/9 to 7/3 and the co-precipitation is conducted together with a basic solution, such as a solution containing NaOH and Na₂CO₃. Particularly, where the catalyst is a Cu-Al spinel the atomic ratio Cu²⁺/Al³⁺ in the Cu-Al solution is 1/2.

### EXAMPLE 1

A fixed bed reactor is charged with 4.0 g as fractionized particles, 0.3-0.7 mm, of copper alumina catalyst (34 wt% Cu, 28 wt% Al, Na and other impurities below 0.5 wt%, with oxygen as balance op to 100 wt%). More specifically, the catalyst is a stoichiometric Cu-Al spinel prepared by a co-precipitation method (P.A. Kumar et al, Journal of Molecular Catalysis A: Chemical 291 (2008) 66-74): a basic solution is prepared from 2M NaOH and 1 M Na₂CO₃ (solution 1) and an aqueous solution of Cu(II) and Al(III) nitrates (solution 2) is prepared. The solutions are taken into separate funnels and are then added dropwise to a beaker containing 1000 mL of distilled water under vigorous stirring. The resulting solution is aged for an hour at 60-70°C with vigorous stirring. It then follows several washings with distilled water to attain neutral pH. Then the solutions is filtered and the resulting precipitate is oven dried for 12, and subsequently calcined at 835°C for 5 hrs in static air.

The catalyst is activated at 370°C for 5 hrs by reducing in 5% H₂ and 95% N₂. A heating ramp of 0.5°C/min is used.

The reactor is heated to 200°C using a hydrogen flow of 200 ml/min at 20 bar pressure (about 20 atm). Furfural vapour is then passed over the catalyst (0.08 ml/min, WHSV is about 1.0 g/g/h). The product vapours are quantified using an online-GC system, and liquid samples are also collected and analyzed by GC-MS to identify the product components. It was found that the product consists of 10 wt% GVL, 10 wt% 2-methyltetrahydrofuran,5 wt% 1-Pentanol and 75 wt% 2-methylfuran.

### EXAMPLE 2

A fixed bed reactor is charged with 4.0 g as fractionized particles, 0.3-0.7 mm, of the same copper alumina catalyst as used in example 1. The catalyst is activated at 370°C for 5 hrs by reducing in 5% H₂ and 95% N₂. A heating ramp of 0.5°C/min is used.

The reactor is heated to 180°C using a hydrogen flow of 200 ml/min at ambient pressure. A solution containing 7 wt.% of furfural in water is vaporized and passed over the catalyst (0.08 ml/min, WHSV is about 0.084 g/g/h). The product vapours are quantified using an online-GC system. The product consists of 21 wt% GVL, 73 wt% 2-methylfuran with the balance being 3 wt% of 2-methyltetrahydrofuran and 3 wt% 1-pentanol.

## Claims

1. Process for the production of gamma-valerolactone (GVL) by conversion in the gas phase of a stream comprising furfural or a stream comprising furfuryl alcohol in the presence of a heterogeneous catalyst comprising copper and alumina in the form of a copper-alumina (Cu-Al) spinel.

2. Process according to claim 1 wherein the stream comprising furfural or stream comprising furfuryl alcohol is co-fed with water.

3. Process according to claims 1 or 2, wherein the copper-alumina spinel is a precipitated stoichiometric Cu-Al spinel (CuAl₂O₄).

4. Process according to any of claims 1 to 3, wherein the catalyst is prepared by a co-precipitation method comprising the use of a Cu and Al solution.

5. Process according to claim 4, wherein the atomic ratio Cu²⁺/Al³⁺ in the Cu and Al solution is in the range 1/9 to 7/3 and the co-precipitation is conducted together with a basic solution.

6. Process according to any of claims 1 to 5, wherein the process is conducted at a reaction temperature in the range 100 to 300°C.

7. Process according to any of claims 1 to 5, wherein the process is conducted at a reaction temperature in the range 120-190°C.

8. Process according to any of claims 1 to 5, wherein the process is conducted at a reaction temperature in the range 160-180°C.

9. Process according to any of claims 1 to 8, wherein the reaction pressure is in the range 1-30 atm.

10. Process according to any of claims 1 to 8, wherein the reaction pressure is in the range 10-25 atm.

11. Process according to any of claims 1 to 10, wherein furfural is derived from C5-sugars.

12. Process according to any of claims 1 to 11 further comprising converting GVL to liquid hydrocarbons.

## Patentansprüche

1. Verfahren zur Herstellung von gamma-Valerolacton (GVL) durch Umsetzen eines Stroms enthaltend Furfural oder eines Stroms enthaltend Furfurylalkohol in der Gasphase in Gegenwart eines heterogenen Katalysators, umfassend Kupfer und Aluminiumoxid in Form eines Kupfer-Aluminiumoxid (Cu-Al)-Spinells.

2. Verfahren gemäß Anspruch 1, wobei dem Strom enthaltend Furfural oder dem Strom enthaltend Furfurylalkohol Wasser zugeführt wird.

3. Verfahren gemäß Anspruch 1 oder 2, wobei der Kupfer-Aluminiumoxid - Spinell ein gefällter stöchiometrischer Cu-Al - Spinell (CuAl₂O₄) ist.

4. Verfahren gemäß einem der Ansprüche 1 bis 3, wobei der Katalysator durch ein Kopräzipitationsverfahren hergestellt wird, umfassend die Verwendung einer Cu- und AI-Lösung.

5. Verfahren gemäß Anspruch 4, wobei das Atomverhältnis Cu²⁺/Al³⁺ in der Cu- und AI-Lösung im Bereich von 1/9 bis 7/3 liegt und die Kopräzipitation zusammen mit einer basischen Lösung durchgeführt wird.

6. Verfahren gemäß einem der Ansprüche 1 bis 5, wobei das Verfahren bei einer Reaktionstemperatur im Bereich von 100 bis 300°C durchgeführt wird.

7. Verfahren gemäß einem der Ansprüche 1 bis 5, wobei das Verfahren bei einer Reaktionstemperatur im Bereich von 120-190°C durchgeführt wird.

8. Verfahren gemäß einem der Ansprüche 1 bis 5, wobei das Verfahren bei einer Reaktionstemperatur im Bereich von 160-180°C durchgeführt wird.

9. Verfahren gemäß einem der Ansprüche 1 bis 8, wobei der Reaktionsdruck im Bereich von 1-30 atm liegt.

10. Verfahren gemäß einem der Ansprüche 1 bis 8, wobei der Reaktionsdruck im Bereich von 10-25 atm liegt.

11. Verfahren gemäß einem der Ansprüche 1 bis 10, wobei Furfural aus C₅-Zuckern bezogen wird.

12. Verfahren gemäß einem der Ansprüche 1 bis 11, weiter umfassend eine Umwandlung von GVL in flüssige Kohlenwasserstoffe.

## Revendications

1. Procédé pour la production de gamma-valérolactone (GVL) par conversion en phase gazeuse d'un courant comprenant du furfural ou d'un courant comprenant de l'alcool furfurylique en présence d'un catalyseur hétérogène comprenant du cuivre et de l'alumine sous la forme d'un spinelle de cuivre-alumine (Cu-AI).

2. Procédé selon la revendication 1, dans lequel le courant comprenant du furfural ou le courant comprenant de l'alcool furfurylique est alimenté de manière conjointe avec de l'eau.

3. Procédé selon la revendication 1 ou 2, dans lequel le spinelle de cuivre-alumine est un spinelle précipité stoechiométrique de Cu-AI (CuAl₂O₄).

4. Procédé selon l'une quelconque des revendications 1 à 3, dans lequel on prépare le catalyseur via un procédé de coprécipitation comprenant l'utilisation d'une solution de Cu et de Al.

5. Procédé selon la revendication 4, dans lequel le rapport atomique Cu²⁺/Al³⁺ dans la solution de Cu et de Al se situe dans la plage de 1/9 à 7/3 et la coprécipitation est mise en oeuvre de manière conjointe avec une solution basique.

6. Procédé selon l'une quelconque des revendications 1 à 5, dans lequel on met en oeuvre le procédé à une température de la réaction dans la plage de 100 à 300°C.

7. Procédé selon l'une quelconque des revendications 1 à 5, dans lequel on met en oeuvre le procédé à une température de la réaction dans la plage de 120-190°C.

8. Procédé selon l'une quelconque des revendications 1 à 5, dans lequel on met en oeuvre le procédé à une température de la réaction dans la plage de 160-180°C.

9. Procédé selon l'une quelconque des revendications 1 à 8, dans lequel la pression de la réaction se situe dans la plage de 1-30 atm.

10. Procédé selon l'une quelconque des revendications 1 à 8, dans lequel la pression de la réaction se situe dans la plage de 10-25 atm.

11. Procédé selon l'une quelconque des revendications 1 à 10, dans lequel le furfural dérive de sucres en C₅.

12. Procédé selon l'une quelconque des revendications 1 à 11, comprenant en outre la conversion de la GVL en hydrocarbures liquides.
